# EUROPEAN PATENT SPECIFICATION

(11) **EP 2 696 822 B1**
(45) Date of publication and mention of the grant of the patent: **15.07.2015**
(21) Application number: 12713159.7
(22) Date of filing: 11.04.2012
(51) Int. Cl.: A61F 9/00

(54) **APPARATUS FOR INTRAOCULAR INJECTION**
VORRICHTUNG ZUR INTRAOKULAREN INJEKTION
APPAREIL POUR INJECTION INTRAOCULAIRE

(30) Priority: 13.04.2011 EP 11162334
(43) Date of publication of application: 19.02.2014
(73) Proprietor: Sanofi-Aventis Deutschland GmbH, 65929 Frankfurt am Main (DE)
(72) Inventor: CLARKE, Alastair Robert, Nantwich Cheshire CW5 6JP (GB); VAUGHAN, Ross, Crewe Cheshire CW4 8BE (GB); HEIGHTON, David, Crescent Wormit Fife DD6 8PU (GB)
(74) Representative: Keil & Schaafhausen Patent- und Rechtsanwälte
(86) International application number: PCT/EP2012/056587
(87) International publication number: WO 2012/140088

(56) References cited:
- WO-A1-2010/077136
- US-A- 4 981 479
- US-A1- 2002 032 415

## Description

The present invention relates to an apparatus for intraocular injection and a system comprising the apparatus and an injection device. An intraocular injection is used to treat eyes, such as eyes of mammals having eye disorders or diseases.

### Background

A number of vision-threatening disorders or diseases of the eye need to deliver a drug (medicament or proteins or the like) by intraocular delivery (more specifically intravitreal delivery), especially when it is useful to deliver high concentrations of drugs. One such technique for intraocular delivery is accomplished by intraocular injection of the drug or capsules containing the drug directly into the vitreous body or by locating a device or capsule containing the drug in the vitreous with a syringe. Such an operation is used in particular for injection of compositions in the vitreous body of the eye in order to treat diseases affecting the retina or choroid, or ciliary body or the lens.

After delivery of drugs to the interior of the eye, such as the vitreous body, it is desirable that a point of entry of any drug delivery device closes and heals or seals as quickly and completely as possible after withdrawal of the drug delivery device. Sealing prevents reflux of the delivered drug, reduces internal eye pressure, heals the eye tissue affected (e.g. sclera), and prevents infections and other complications.

The document WO 2010/077136 A1 shows and describes an ophthalmic device and an intravitreal method wherein the ophthalmic device administers a substance into a human or animal eye by means of a needle. The device comprises a support structure having a lower surface for contacting the conjunctiva of a patient's eye. Further, the support structure comprises a substantially tapering part directed away from the lower surface. The structure also comprises a substantially straight channel in the tapering part. The orientation of the channel is perpendicular to the lower surface that makes contact with the eye in order to secure that the needle penetrates the sclera perpendicularly.

An apparatus for intraocular injection is known from documents WO 2008/084063 A1 and WO 2008/084064 A1. These documents describe a technique wherein the superficial layer of the eye (conjunctiva) is urged to slide over the underlying layer (sclera) by a flexible leg of a resilient member during a downward movement of the whole apparatus into the direction of the eye so that the layers are shifted one relative to the other prior to the needle penetrating into the eye. When the injection apparatus and hence the resilient member are removed from the eye, the superficial layer, i.e. the conjunctiva, slides over the underlying layer (sclera) back to its initial position.

The known apparatus is constructed in the way that the flexible leg is the first portion of the apparatus to come into contact with the eye. Thus, if the leg does not grip the superficial layer of the eye or simply flexes without causing dis-placement of the superficial layer, the desired displacement of the superficial layer over the underlying layer will not be achieved. Further, during downward movement of the known apparatus, the placement of the apparatus may be imprecise and therefore the point of insertion of the needle may be incorrect. However, it is important to exactly find the right position for puncturing the eye in order to avoid damaging structures located in front or in the rear of the vitreous body. The known apparatus may tend to slide away from the desired point of insertion.

It is therefore an object of the present invention to provide an apparatus for intraocular injection which could precisely be positioned in a desired zone of the eye and would allow for displacement of the superficial layer of the eye relative to the underlying layer prior to drug delivery and return of the superficial layer to its original position after drug delivery to allow for, e.g., occlusion of the point of entry of the drug delivery device. Accordingly, a corresponding method is pre-sented.

This problem is solved with an apparatus having the features of claim 1.

### Summary of the Invention

The present invention relates to apparatuses for intraocular injection. In an exemplary embodiment, the apparatus comprises a body adapted to accommo-date an injection device, a displacement device coupled to a distal end of the body, and a sleeve coupled to the body and axially moveable relative to the body. The displacement device includes at least one rotatable member adapted to contact a superficial layer of an eye, and the sleeve engages and causes rotation of the at least one rotatable member as the sleeve moves from a first axial position to a second axial position. Rotation of the at least one rotatable member displaces the superficial layer relative to an underlying layer of the eye.

The at least one rotatable member may be at least one wheel. The sleeve may engage the at least one rotatable member by frictional contact. A distal end of the sleeve may comprise a tapered section. Movement of the injection device within the body causes movement of the sleeve between the first and second axial positions. The rotation of the at least one rotatable member is limited to a predefined angular rotation.

The at least one rotatable member may be made of at least one of a polymer, silicon, a silicone hydrogel, glass, PMMA, metal and a metal alloy.

The injection device may comprise a syringe. A locking mechanism may be utilized for locking the sleeve in the second position. The sleeve is in the sec-ond position prior to the injection device piercing the superficial layer.

The at least one rotatable member includes a first wheel and a second wheel adapted to rotate in opposing rotational directions when the sleeve moves from the first axial position to the second axial position.

These as well as other advantages of various aspects of the present invention will become apparent to those of ordinary skill in the art by reading the following detailed description, with appropriate reference to the accompanying drawings.

### Brief Description of the Drawings

Exemplary embodiments are described herein with reference to the schematic drawings in which:
Fig. 1 illustrates an exemplary embodiment of an apparatus for intraocular injection in a general cross section prior to dis-placement of conjunctiva and delivery of injection;
Fig. 2 shows the cross section of Fig. 1 after conjunctiva displace-ment and needle insertion;
Figures 3 and 4 show a central view of the cross section depicted in Fig. 2; and
Fig. 5 illustrates a perspective view of an exemplary embodiment of a displacement device of the embodiment shown in Fig-ures 1 to 4.

### Detailed Description

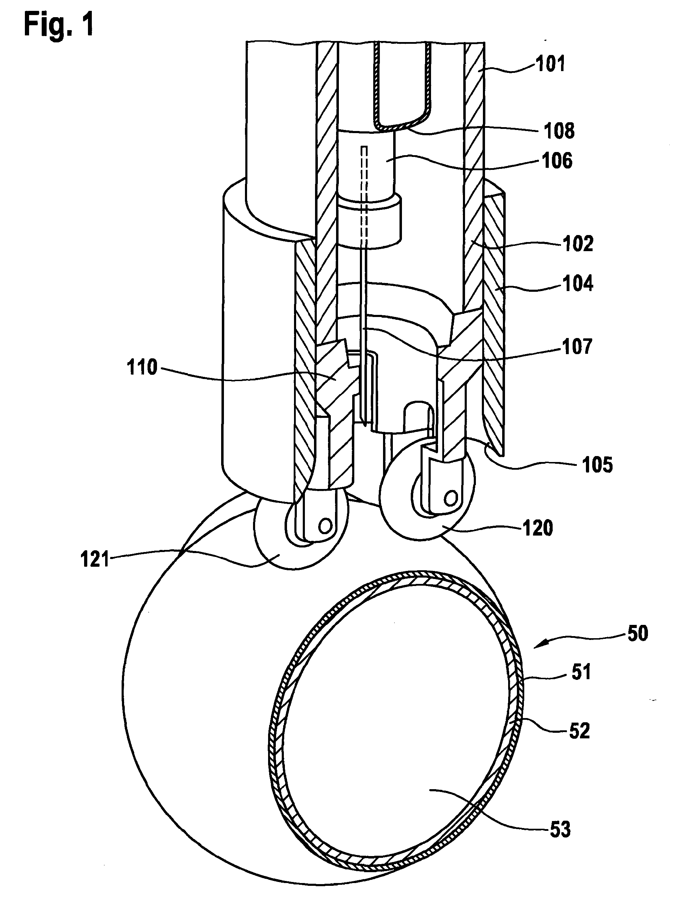

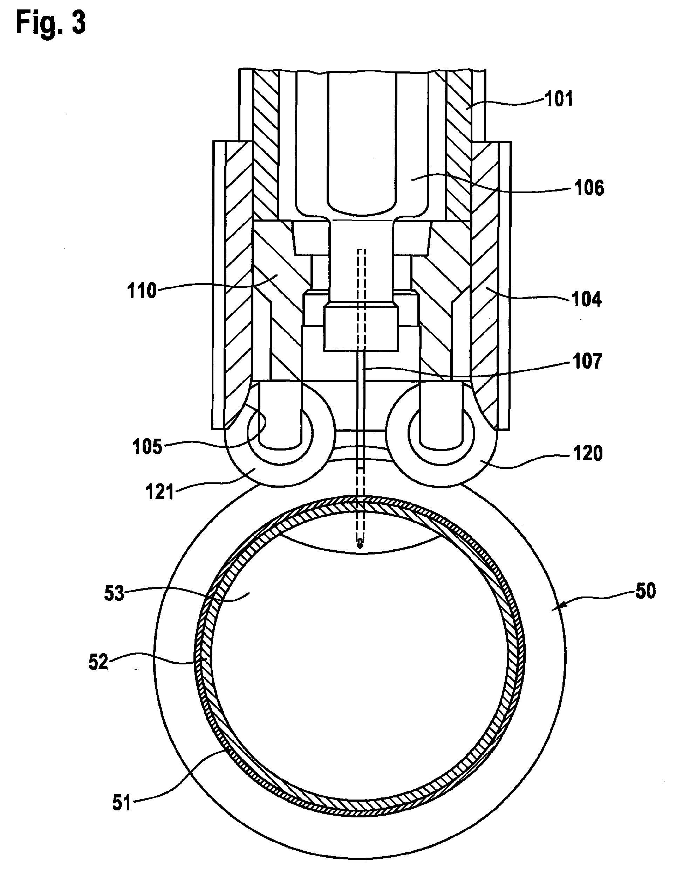

Figures 1 to 5 illustrate exemplary embodiments of an apparatus for intraocular injection comprising a body 101, which may be formed as a hollow tube to ac-commodate an injection device, e.g., a syringe 106. The body 101 may include a proximal section (not shown) and a distal section 102. The distal section 102 may be surrounded by a sleeve 104, which in an exemplary embodiment, fits telescopically on the distal section 102 of body 101 and is moveable between retracted and extended positions. In another exemplary embodiment, the sleeve 104 may surround all or more of the body 101 than the distal section 102. In yet another exemplary embodiment, the sleeve 104 may be disposed and axially moveable within the distal section 102 of the body 101.

In an exemplary embodiment, the body 101 is sized and shaped to receive a syringe 106 comprising a needle 107, a neck 108 and a barrel or cartridge which holds a medicament. The body 101 and the syringe 106 may be separate com-ponents or formed as a single device (e.g., a user does not have access to the syringe 106). In case that body 101 and syringe 106 are separate components, the apparatus may be re-usable and the syringe or cartridge may be exchange-able and/or re-fillable.

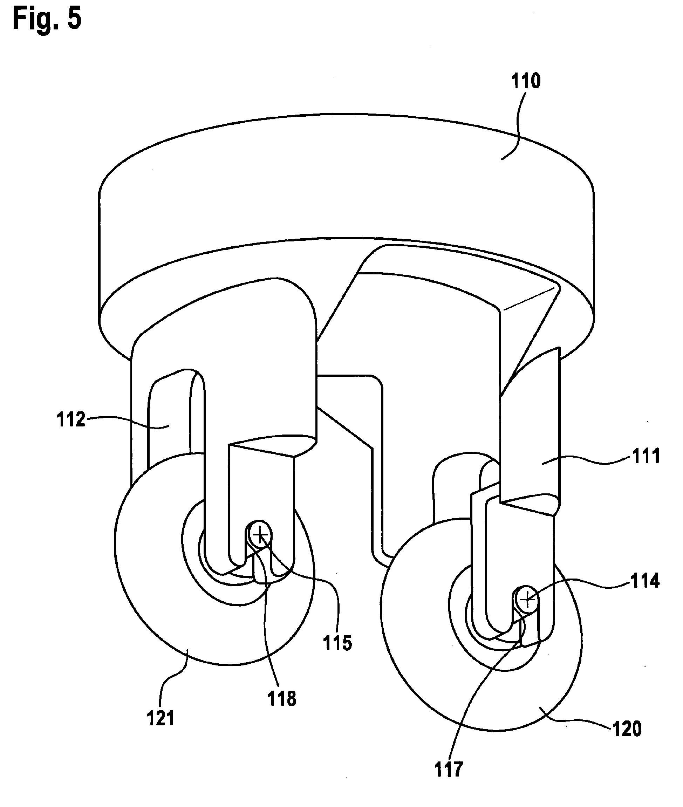

A conjunctiva displacement device 110 may be located at a distal end of the body 101. An exemplary embodiment of the conjunctiva displacement device 110 is shown in Fig. 5 in detail. The displacement device 110 may be integrally formed with the distal end of the body 101 or removably coupled thereto (e.g., via threaded fit, snap fit, bayonet fit or friction fit). In an embodiment in which the displacement device 110 is removably coupled to the body 101, a proximal end of the displacement device 110 may include a coupling mechanism, e.g., threads, snaps, clamps, hooks, etc. for mating with a corresponding coupling mechanism formed on the distal end of the body 101.

In the exemplary embodiment shown in Fig. 5, a distal end of the displacement device 110 includes a first fork 111 comprising a first axis 114 guided in a first bearing 117 and a second fork 112 comprising a second axis 115 guided in a second bearing 118. In the exemplary arrangement, the first and second forks 111, 112 are substantially parallel to a longitudinal axis of the body 101, and the first and second axes 114, 115 are substantially perpendicular to the first and second forks 111, 112, respectively. A first wheel 120 is rotatably mounted on the first axis 114, and a second wheel 121 is rotatably mounted on the second axis 115. In use, the wheels 120, 121 contact a superficial layer (conjunctiva 51) of the eye 50. While the exemplary embodiment depicts a displacement device 110 with two wheels, those of skill in the art will understand that one or more wheels may be used, and that the wheels may be replaced with objects having other shapes (e.g., cylinders, cones, ellipses) or partial shapes (e.g., arcs, U-shaped components).

In an exemplary embodiment, the apparatus may be utilized to administer a drug or the like into an eye, e.g. a vitreous body 53. Prior to use, the needle 107 of the syringe 106 may be contained within the body 101, e.g., to prevent injury, and a distal opening of the body 101 and/or the sleeve 104 may be covered with a film to maintain sterility of the needle 107.

In use of the inventive apparatus, a physician separates eye lids of a patient using an eye lid retractor. The apparatus may then be aligned on the eye to ensure that an injection site will not pierce the cornea or limbus, but be directed into the vitreous 53. For example, the wheels 120, 121 and/or the sleeve 104 may be used to align with the cornea or another anatomical feature of the eye or surrounding anatomy to ensure that the injection site will not pierce the cornea or limbus, but be directed into the vitreous 53. Those of skill in the art will un-derstand that the sleeve 104 and/or body 101 may be made from an at least partially transparent material such that alignment may be facilitated. When the apparatus is being positioned for an injection, the sleeve 104 may be in the retracted position to allow the physician to visualize the placement of the apparatus and potential injection site.

When the apparatus has been properly placed on the eye 50, the wheels 120, 121 contact the conjunctiva 51 as shown in Fig. 1. In other words, the complete apparatus is offered up to the patient's eye such that the wheels 120, 121 are in contact with the conjunctiva layer 51.

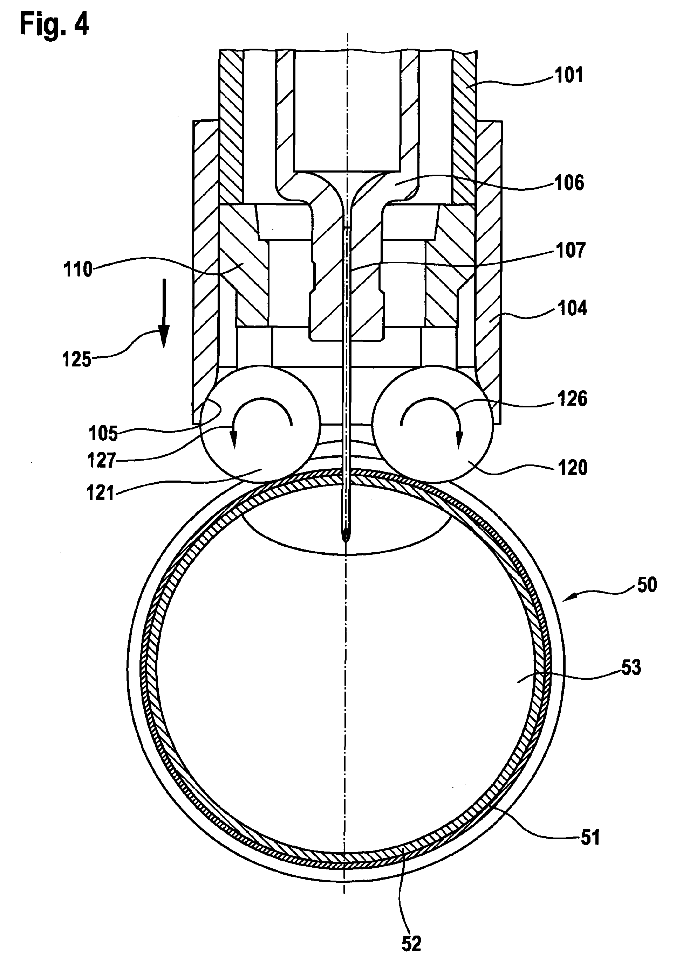

A physician may depress a plunger or similar depressible or moveable element coupled to the body 101 and/or the syringe 106 which advances the syringe 106 distally within the body 101 towards the injection site. As the syringe 106 moves distally within the body 101 it, the axial movement of the syringe 106 may also drive the sleeve 104 distally (see arrow 125 in Fig. 4) until the sleeve 104 reaches its extended position.

In one exemplary embodiment, a cuff may coupled to or adjacent the neck 108 or barrel of the syringe 106. The cuff may be attached to the sleeve 104 (e.g., through one or more arms extending through slots formed in the body 101). As the syringe 106 moves distally within the body 101, the syringe 106 forces the cuff (and resultantly, the sleeve 104) to move distally, as well. The cuff may include a spring, which biases the syringe 106 in a retracted position within the body 101, such that after administration of the injection, the spring force causes the syringe 106 to be returned to the retracted position.

In another exemplary embodiment, the sleeve 104 may be moved manually by a user. For example, the apparatus may be positioned on the eye 50, and the user may move the sleeve 104 into an extended position prior to the injection and back to the retracted position after the injection. Or, the sleeve 104 may remain in the extended position after the injection to prevent exposure of the needle 107.

In an exemplary embodiment, a locking mechanism may also be utilized to prevent the sleeve 104 from returning to the retracted position after it has been moved into the extended position.

As the sleeve 104 moves from the retracted position into the extended position, an inner surface, e.g., tapered section 105, of the distal end of the sleeve 104 contacts an outer surface of the wheels 120, 121. Further distal movement of sleeve 104 causes the wheels 120, 121 to rotate by their contact with the ta-pered section 105. The rotation of the wheels 120, 121 may be limited by, for example, a projection formed on each of the wheels 120, 121 parallel to the axes 114, 115 which abuts the respective forks 111, 112 after a predefined angular rotation. In another exemplary embodiment, rotation of the wheels 120, 121 may be limited by an angle of the tapered section 105 relative to an outer surface of the sleeve 104. For example, as the angle of the tapered section 105 relative to the outer surface of the sleeve 104 increases, a limit of the angular rotation of the wheels 120, 121 may increase.

As shown in the exemplary embodiment in Fig. 4, distal movement of the sleeve 104 causes the wheels 120, 121 to rotate in opposite directions (see arrows 126, 127), displacing the conjunctiva 51 (relative to the sclera 52) toward the injection site. Those of skill in the art will understand that various modifications may be made to the shape of the sleeve 104 to effect different directional and magnitude of displacement of the conjunctiva 51 relative to the sclera 52. For example, the sleeve 104 may be shaped such that distal movement of the sleeve 104 causes the wheels 120, 121 to rotate in the same the direction.

The surface of the wheels 120, 121 may partly or fully consist of a polymer, silicon, a silicone hydrogel, glass, PMMA, metal, metal alloy or any other mate-rial which is not harmful to the conjunctiva 51 but could provide a frictional hold on the conjunctiva 51 for displacing it relative to the sclera 52. In an exemplary embodiment, at least one of the wheels 120, 121 may have a textured surface to ensure that the conjunctiva 51 will be displaced upon rotation of the wheels 120, 121.

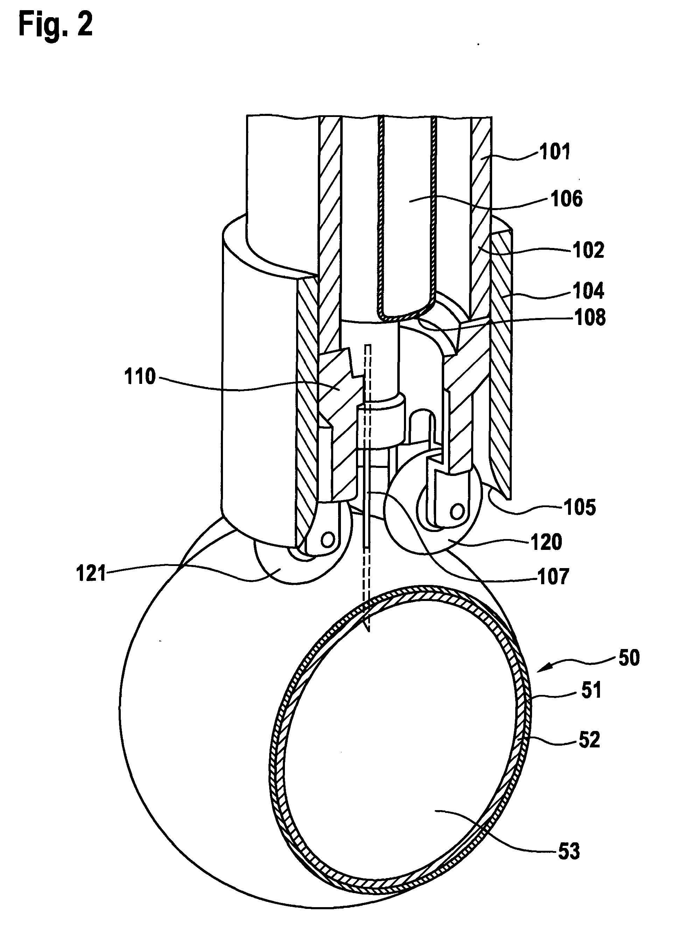

The situation where the needle 107 punctures the eye 50 is illustrated in Figs. 2-4. In an exemplary embodiment, after the conjunctiva 51 has been displaced relative to the sclera 52 by the rotation of the wheels 120, 121, the syringe 106 moves further distally within the body 101 and the needle 107 penetrates the displaced conjunctiva 51, then the sclera 52 and after that it penetrates into the vitreous body 53 of the eye 50. In this position, the drug or the like contained within the syringe 106 is administered into the vitreous body 53 (intravitreal injection). Examples of such a drug may include, but are not limited to, steroids or monoclonal antibodies used, for example, to treat macular degeneration. Those of skill in the art will understand that various medicaments and/or thera-peutic substances and/or implantable devices may be administered using the apparatus.

In an exemplary embodiment, after dispensing the medicament, the apparatus is removed from the eye 50. The syringe 106 may be withdrawn into the body 101, e.g., by a spring or gearing mechanism. In an exemplary embodiment, the sleeve 104 may be returned to its retracted position, rotating the wheels in direc-tions opposition those shown in Fig. 4 and causing the conjunctiva 51 to return to its original position. In another exemplary embodiment, the apparatus may be removed from the eye 50 with the sleeve 104 in its extended position such that the sleeve 104 shields the needle 107, preventing a needle stick injury. In this exemplary embodiment, the conjunctiva 51 may be returned to its original posi-tion due to the elastic nature of the tissue forming the conjunctiva 51.

Thus, use of the apparatus creates a punctured region (orifice) of the conjunctiva 51 that is offset to the punctured region (orifice) of sclera 52, when the conjunctiva 51 is returned to its pre-injection position. Hence, after the injection is complete, the conjunctiva 51 seals the orifice of sclera 52 which may prevent reflux of the delivered drug, reduce the effects of the procedure on the internal eye pressure, assist with the healing of the eye 50 and reduces the risk of infection.

Those of skill in the art will understand that modifications (additions and/or removals) of various components of the apparatuses, methods and/or systems and embodiments described herein may be made without departing from the full scope the present invention.

### Reference numbers:

- 50: eye
- 51: conjunctiva
- 52: sclera
- 53: vitreous body
- 101: body
- 102: distal section
- 104: sleeve
- 105: tapered section at the inner side of sleeve 104
- 106: syringe
- 107: needle
- 108: neck of syringe
- 110: conjunctiva displacement device
- 111: first fork
- 112: second fork
- 114: first axis of first wheel 120
- 115: second axis of second wheel 121
- 117: first bearing of first wheel 120
- 118: second bearing of second wheel 121
- 120: first wheel
- 121: second wheel
- 125, 126, 127: arrow

## Claims

1. An apparatus for intraocular injection comprising
a body (101) adapted to accommodate an injection device;
a sleeve (104) coupled to the body (101) and axially moveable relative to the body (101);
a displacement device (110) coupled to a distal end (102) of the body (101),
**characterized in that** the displacement device (110) includes at least one rotatable member (120, 121) adapted to contact a superficial layer (51) of an eye (50);
and **in that** the sleeve (104) engages and causes rotation of the at least one rotatable member (120, 121) as the sleeve (104) moves from a first axial position to a second axial position, wherein the rotation of the at least one rotatable member (120, 121) displaces the superficial layer (51) relative to an underlying layer (52) of the eye (50).

2. The apparatus according to claim 1 wherein the at least one rotatable member is at least one wheel (120, 121).

3. The apparatus according to any of the preceding claims wherein the sleeve (104) engages the at least one rotatable member (120, 121) by frictional contact.

4. The apparatus according to claim 1 wherein a distal end of the sleeve (104) comprises a tapered section (105).

5. The apparatus according to any of the preceding claims wherein movement of the injection device within the body (101) causes movement of the sleeve (104) between the first and second axial positions.

6. The apparatus according to any of the preceding claims wherein the rotation of the at least one rotatable member (120, 121) is limited to a predefined angular rotation.

7. The apparatus according to any of the preceding claims wherein the at least one rotatable member (120, 121) is at least partially made of at least one of a polymer, silicon, a silicone hydrogel, glass, PMMA, metal and a metal alloy.

8. The apparatus according to any of the preceding claims wherein the injection device comprises a syringe (106).

9. The apparatus according to any of the preceding claims further including a locking mechanism for locking the sleeve (104) in the second position.

10. The apparatus according to any of the preceding claims wherein the at least one rotatable member includes a first wheel (120) and a second wheel (121).

11. The apparatus according to claim 10, wherein the first wheel (120) and the second wheel (121) are adapted to rotate in opposing rotational directions when the sleeve (104) moves from the first axial position to the second axial position.

12. A system comprising the apparatus according to any of the preceding claims and an injection device, wherein the injection device is accommodated by the body of the apparatus and the system is adapted such that the sleeve (104) is moved in the second axial position prior to the injection device piercing the superficial layer (51).

## Patentansprüche

1. Vorrichtung zur intraokularen Injektion, umfassend:
einen zur Aufnahme einer Injektionsvorrichtung ausgelegten Körper (101);
eine mit dem Körper (101) verbundene und relativ zu dem Körper (101) axial bewegbare Hülse (104);
eine mit dem distalen Ende (102) des Körpers (101) verbundene Verschiebevorrichtung (110),
**dadurch gekennzeichnet, dass** die Verschiebevorrichtung (110) zumindest ein drehbares Element (120, 121) aufweist, das für den Kontakt mit einer Oberflächenschicht (51) eines Auges (50) ausgelegt ist; und dass die Hülse (104) in das zumindest eine drehbare Element (120, 121) eingreift und eine Drehung desselben verursacht, wenn sich die Hülse (104) aus einer ersten axialen Stellung in eine zweite axiale Stellung bewegt, wobei die Drehung des zumindest einen drehbaren Elements (120, 121) die Oberflächenschicht (51) relativ zu einer darunter liegenden Schicht (52) des Auges (50) verschiebt.

2. Vorrichtung nach Anspruch 1, wobei das zumindest eine drehbare Element zumindest ein Rad (120, 121) ist.

3. Vorrichtung nach einem der vorhergehenden Ansprüche, wobei die Hülse (104) durch reibschlüssigen Kontakt in das zumindest eine drehbare Element (120, 121) eingreift.

4. Vorrichtung nach Anspruch 1, wobei ein distales Ende der Hülse (104) einen verjüngten Abschnitt (105) umfasst.

5. Vorrichtung nach einem der vorhergehenden Ansprüche, wobei eine Bewegung der Injektionsvorrichtung in dem Körper (101) eine Bewegung der Hülse (104) zwischen den ersten und zweiten axialen Stellungen verursacht.

6. Vorrichtung nach einem der vorhergehenden Ansprüche, wobei die Drehung des zumindest einen drehbaren Elements (120, 121) auf eine vorab festgelegte Winkeldrehung beschränkt ist.

7. Vorrichtung nach einem der vorhergehenden Ansprüche, wobei das zumindest eine drehbare Element (120, 121) zumindest teilweise zumindest aus einem Polymer, Silikon, Silikonhydrogel, Glas, PMMA, Metall oder einer Metalllegierung hergestellt ist.

8. Vorrichtung nach einem der vorhergehenden Ansprüche, wobei die Injektionsvorrichtung eine Spritze (106) umfasst.

9. Vorrichtung nach einem der vorhergehenden Ansprüche, ferner einen Arretierungsmechanismus zur Arretierung der Hülse (104) in der zweiten Stellung aufweisend.

10. Vorrichtung nach einem der vorhergehenden Ansprüche, wobei das zumindest eine drehbare Element ein erstes Rad (120) und ein zweites Rad (121) aufweist.

11. Vorrichtung nach Anspruch 10, wobei das erste Rad (120) und das zweite Rad (121) ausgelegt sind, sich in entgegengesetzte Drehrichtungen zu drehen, wenn sich die Hülse (104) aus der ersten axialen Stellung in die zweite axiale Stellung bewegt.

12. System, umfassend die Vorrichtung nach einem der vorhergehenden Ansprüche und eine Injektionsvorrichtung, wobei die Injektionsvorrichtung von dem Körper der Vorrichtung aufgenommen wird und das System so ausgelegt ist, dass die Hülse (104) in die zweite axiale Stellung bewegt wird, bevor die Injektionsvorrichtung in die Oberflächenschicht (51) einsticht.

## Revendications

1. Appareil d'injection intraoculaire comprenant :
un corps (101) conçu pour recevoir un dispositif d'injection ;
un manchon (104) accouplé au corps (101) et mobile axialement par rapport au corps (101) ;
un dispositif de déplacement (110) accouplé à une extrémité distale (102) du corps (101),
**caractérisé en ce que** le dispositif de déplacement (110) comprend au moins un élément rotatif (120, 121) conçu pour venir en contact avec une couche superficielle (51) d'un oeil (50) ;
et **en ce que** le manchon (104) vient en prise avec le ou les éléments rotatifs (120, 121) et entraîne sa/leur rotation à mesure que le manchon (104) se déplace d'une première position axiale à une seconde position axiale, la rotation du ou des éléments rotatifs (120, 121) déplaçant la couche superficielle (51) par rapport à une couche sous-jacente (52) de l'oeil (50).

2. Appareil selon la revendication 1, dans lequel le ou les éléments rotatifs est/sont au moins une roue (120, 121).

3. Appareil selon l'une quelconque des revendications précédentes, dans lequel le manchon (104) vient en prise avec le ou les éléments rotatifs (120, 121) par contact de frottement.

4. Appareil selon la revendication 1, dans lequel une extrémité distale du manchon (104) comprend une section décroissante (105).

5. Appareil selon l'une quelconque des revendications précédentes, dans lequel le déplacement du dispositif d'injection à l'intérieur du corps (101) entraîne un déplacement du manchon (104) entre les première et seconde positions axiales.

6. Appareil selon l'une quelconque des revendications précédentes, dans lequel la rotation du ou des éléments rotatifs (120, 121) est limitée à une rotation angulaire prédéfinie.

7. Appareil selon l'une quelconque des revendications précédentes, dans lequel le ou les éléments rotatifs (120, 121) est/sont au moins partiellement composé(s) d'au moins un matériau parmi un polymère, du silicium, un hydrogel de silicone, du verre, du PMMA, du métal et un alliage métallique.

8. Appareil selon l'une quelconque des revendications précédentes, dans lequel le dispositif d'injection comprend une seringue (106).

9. Appareil selon l'une quelconque des revendications précédentes, comprenant en outre un mécanisme de verrouillage permettant de verrouiller le manchon (104) dans la seconde position.

10. Appareil selon l'une quelconque des revendications précédentes, dans lequel le ou les éléments rotatifs comprend/comprennent une première roue (120) et une seconde roue (121).

11. Appareil selon la revendication 10, dans lequel la première roue (120) et la seconde roue (121) sont conçues pour tourner dans des sens de rotation opposés lorsque le manchon (104) se déplace de la première position axiale à la seconde position axiale.

12. Système comprenant l'appareil selon l'une quelconque des revendications précédentes et un dispositif d'injection, dans lequel le dispositif d'injection est reçu par le corps de l'appareil et le système est conçu de telle sorte que le manchon (104) est déplacé jusqu'à la seconde position axiale avant que le dispositif d'injection ne perfore la couche superficielle (51).
